# EUROPEAN PATENT APPLICATION

(11) **EP 4 790 719 A1**
(43) Date of publication of application: **12.08.2026**
(21) Application number: 26156251.6
(22) Date of filing: 04.02.2026
(51) Int. Cl.: G16H 20/30, A61G 7/002, G16H 40/63, G16H 50/30

(54) **PATIENT SUPPORT APPARATUS WITH CARE ASSISTANCE**

(30) Priority: 05.02.2025 US 202563754167 P
(71) Applicant: Hill-Rom Services, Inc., Batesville, IN 47006-9167 (US)
(72) Inventor: BHAI, Aziz Ali, Batesville, 47006 (US)
(74) Representative: Reddie & Grose LLP

(57) **Abstract**

A patient support apparatus includes a frame having one or more sensors, a deck coupled to the frame, a mattress supported by the deck, and a controller mounted on the frame. The controller captures data of a patient resting on the mattress using the one or more sensors. The controller identifies a behavior of the patient based on the data of the patient. The controller engages the patient in a conversation about the behavior of the patient, the conversation driven by generative artificial intelligence. The controller identifies a condition of the patient based on the conversation, and generates a recommendation to relieve the condition of the patient.

## Description

Hospital beds are specialized pieces of equipment designed to provide comfort, support, and safety to patients during their stay in a medical facility. These beds are adjustable to accommodate various patient needs, offering features such as height adjustment, reclining capabilities, and mobility support. Hospital beds also include safety rails that when deployed prevent patients from exiting the bed to meet safety standards. Some hospital beds are equipped with additional functions like built-in monitoring systems, pressure-relief mattresses to prevent bedsores, and integrated equipment to support patient care. Hospital beds facilitate patient recovery, and can both assist medical staff in their duties and improve patient comfort.

In general terms, the present disclosure relates to a patient support apparatus that is configured to provide care assistance. In one possible configuration, the patent support apparatus engages a patient by using generative artificial intelligence, and generates a recommendation based on the engagement with the patient. Various aspects are described in this disclosure, which include, but are not limited to, the following aspects.

One aspect relates to a patient support apparatus, comprising: a frame having one or more sensors; a deck coupled to the frame, the deck configured to support a mattress; and a controller communicatively coupled to the frame, the controller having at least one processing device, and at least one memory device storing software instructions that, when executed by the at least one processing device, cause the at least one processing device to: capture data of a patient resting on the mattress using the one or more sensors; identify a status of the patient based on the data of the patient; engage the patient about the status by using generative artificial intelligence; and generate a recommendation based on engagement with the patient.

Another aspect relates to a method of providing care assistance, the method comprising: capturing data of a patient resting on a mattress of a patient support apparatus; identifying a status of the patient based on the data of the patient; engaging the patient about the status by using generative artificial intelligence; generating a recommendation based on engagement with the patient; and adjusting one or more settings of the patient support apparatus upon acceptance of the recommendation.

Another aspect relates to a patient support apparatus, comprising: a frame having one or more sensors; a deck coupled to the frame, the deck configured to support a mattress; and a controller communicatively coupled to the frame, the controller having at least one processing device, and at least one memory device storing software instructions that, when executed by the at least one processing device, cause the at least one processing device to: receive a communication from a patient resting on the mattress; engage the patient by using generative artificial intelligence; identify a status of the patient based on engagement with the patient; and generate a recommendation based on the status of the patient.

The invention will now be further described by way of an example with reference to the accompanying drawings, in which:
FIG. 1 is a front isometric view of an example of a patient support apparatus.
FIG. 2 is a rear isometric view of the patient support apparatus of FIG. 1.
FIG. 3 is an isometric view of a patient interface device that can be mounted on the patient support apparatus of FIG. 1.
FIG. 4 schematically illustrates an example of the patient support apparatus of FIG. 1.
FIG. 5 schematically illustrates an example of a method of providing care assistance that can be performed by the patient support apparatus of claim 1.
FIG. 6 illustrates a tablet computer that can attach to the patient interface device of FIG. 3, the tablet computer displaying an example of a recommendation that can be generated in accordance with the operations of the method of FIG. 5.
FIG. 7 illustrates a device displaying an example of an alert that can be generated in accordance with the operations of the method of FIG. 5.
FIG. 8 illustrates a display mounted on the patient support apparatus of FIG. 1 that displays an example of a summary of a conversation between a patient and a generative Al application that can be generated in accordance with the operations of the method of FIG. 5.

The disclosed apparatus at least in preferred embodiments provides a practical application that improves patient care through technological integration of sensors, artificial intelligence, and automated responses in a hospital bed system. This practical implementation addresses specific technological problems in healthcare while providing concrete benefits and improvements to existing patient care systems.

The disclosed patient support apparatus implements a concrete technological solution by utilizing multiple integrated sensors to capture real-time patient data, including load cells that detect patient movement and weight distribution, pressure sensors that detect pressure inside mattress bladders, and position sensors that monitor bed articulation angles. The sensor fusion provides comprehensive monitoring that enables detection of patient statuses, behaviors, and conditions.

The disclosed apparatus at least in preferred embodiments provides a tangible improvement to healthcare technology through its generative AI system that processes the sensor data to engage patients in meaningful conversations. This is not merely an abstract concept, but rather a specific technological implementation that includes microphone and speaker units mounted on the bed's siderails for direct patient interaction. The generative AI system processes audio inputs through specialized language models that can adapt to patient accents and preferences, demonstrating a concrete technical solution to communication challenges in healthcare settings.

The practical application extends to specific automated responses and adjustments. When the system detects patient conditions requiring intervention, it can automatically adjust bed settings, such as repositioning articulated sections of the bed or modifying mattress pressure, while following defined protocols and safety parameters. These automated responses represent concrete physical actions that directly improve patient care and comfort.

The disclosed system at least in preferred embodiments further demonstrates its practical application through integration with existing healthcare systems. For example, the technology described herein interfaces with electronic health records (EHR) systems, admission/discharge/transfer (ADT) systems, and nurse call systems, creating a comprehensive care platform that enhances healthcare delivery through specific technological improvements. This integration enables the system to make informed decisions based on patient medical history and current care protocols.

The system includes specific display implementations that provide visual feedback through mounted displays on the bed's siderails and connected tablet computers. These displays show concrete recommendations and summaries of patient interactions, providing caregivers with actionable information that improves patient care efficiency. The visual interface components represent specific technological implementations that transform the abstract concept of patient care into practical, usable tools for healthcare providers.

The practical application is further demonstrated through the system's ability to generate specific alerts and recommendations based on detected patient conditions. These alerts are communicated through defined channels to caregivers' devices, representing a concrete implementation of the technology that improves response times and patient care outcomes. The system's ability to learn from patient interactions and outcomes demonstrates a practical application that continuously improves its effectiveness through specific technological means.

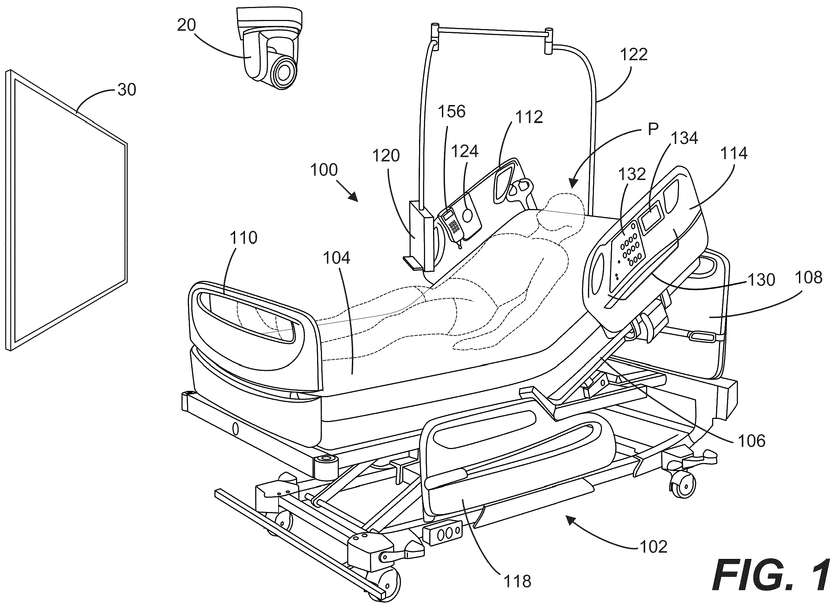

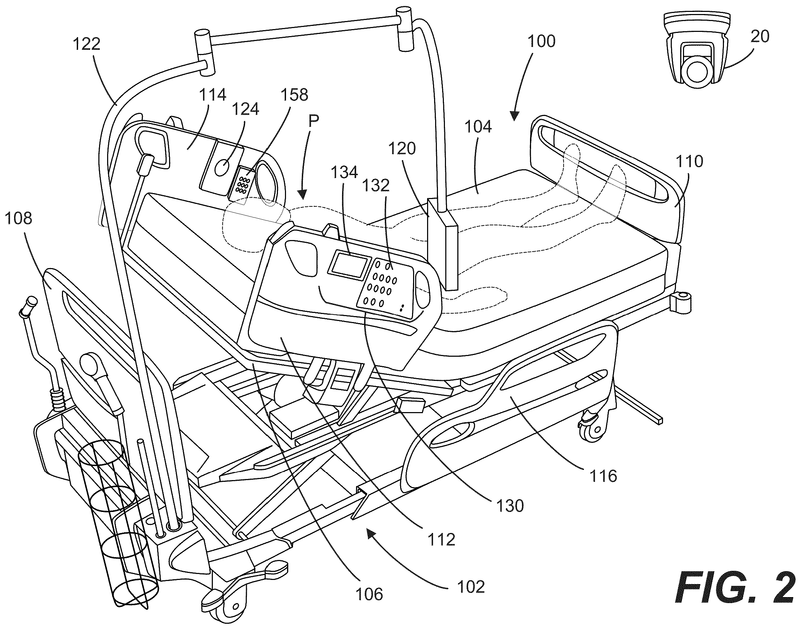

FIG. 1 is a front isometric view of an example of a patient support apparatus 100. FIG. 2 is a rear isometric view of an example of a patient support apparatus 100. The patient support apparatus 100 monitors a patient P who is resting on a mattress 104 supported by a frame 102 of the patient support apparatus 100. The patient support apparatus 100 is further configured to interact with the patient P to provide care assistance such as when a caregiver is not present. The interactions with the patient P are driven by generative artificial intelligence to enhance the effectiveness of the care assistance provided by the patient support apparatus 100.

Artificial intelligence is used to detect interactions and/or patterns of interactions by the patient P with the patient support apparatus 100, and the artificial intelligence recommends adjustment of one or more settings of the patient support apparatus 100 to improve the comfort and/or healthcare of the patient. The patient support apparatus 100 can automatically adjust the one or more settings based on the recommendation. Alternatively, the patient support apparatus 100 adjusts the one or more settings following approval received from the patient P and/or from an authorized user of the patient support apparatus 100 such as a nurse.

In the example shown in FIGS. 1 and 2, the patient support apparatus 100 is depicted as a hospital bed. In alternative examples, the patient support apparatus 100 can include a stretcher, a surgical table, or other structures configured to support a patient within a healthcare environment such as a hospital, nursing home, long term care facility, and the like.

The patient support apparatus 100 includes the frame 102, a deck 106 coupled to the frame 102, and the mattress 104 positioned on the deck 106. The patient support apparatus 100 includes a headboard 108 coupled to the frame 102 and a footboard 110 coupled to the deck 106. The frame 102 is configured to raise and lower the deck 106 relative to the floor to adjust the height of the mattress 104. The frame 102 is further configured to adjust relative angles between articulable sections of the deck 106 such as between a head section, a seat section, and a foot section that are hinged or pivotally coupled together such that relative angles between the head, seat, and foot sections are adjustable to adjust the contour and/or orientation of the mattress 104. For example, the head, seat, and foot sections of the deck 106 can be controlled to position the mattress 104 in a Trendelenburg position or a reverse Trendelenburg position.

The patient support apparatus 100 further includes a plurality of siderails such as a pair of upper siderails coupled to the deck 106 and a pair of lower siderails coupled to the frame 102. The pair of upper siderails include a left upper siderail 112 and a right upper siderail 114. The pair of lower siderails include a left lower siderail 116 and a right lower siderail 118.

In the example shown in FIGS. 1 and 2, the upper siderails each include a control panel 130 that includes controls 132 for adjusting one or more settings of the patient support apparatus 100 such as to adjust the height of the mattress 104, the relative angles of the head section, seat section, and foot section of the deck 106, the firmness of the mattress 104, to raise the siderails to prevent the patient P from exiting from the patient support apparatus 100, or to lower the siderails to allow the patient P to exit the patient support apparatus 100.

The control panel 130 further includes a display 134 that displays information. The display 134 can include a touchscreen that receives inputs from authorized users of the patient support apparatus 100 such as physicians, registered nurses (RN), and other healthcare professionals. The display 134 can use various technologies to sense the inputs from the authorized users such as capacitance to detect changes in an electrical field when a conductive object (e.g., finger or stylus) touches the display 134 to select an object displayed thereon.

The upper siderails can further include microphone and speaker units 124. In some examples, only one of the left upper siderail 112 or the right upper siderail 114 includes a microphone and speaker unit 124. In other examples, both the left upper siderail 112 and the right upper siderail 114 include a microphone and speaker unit 124.

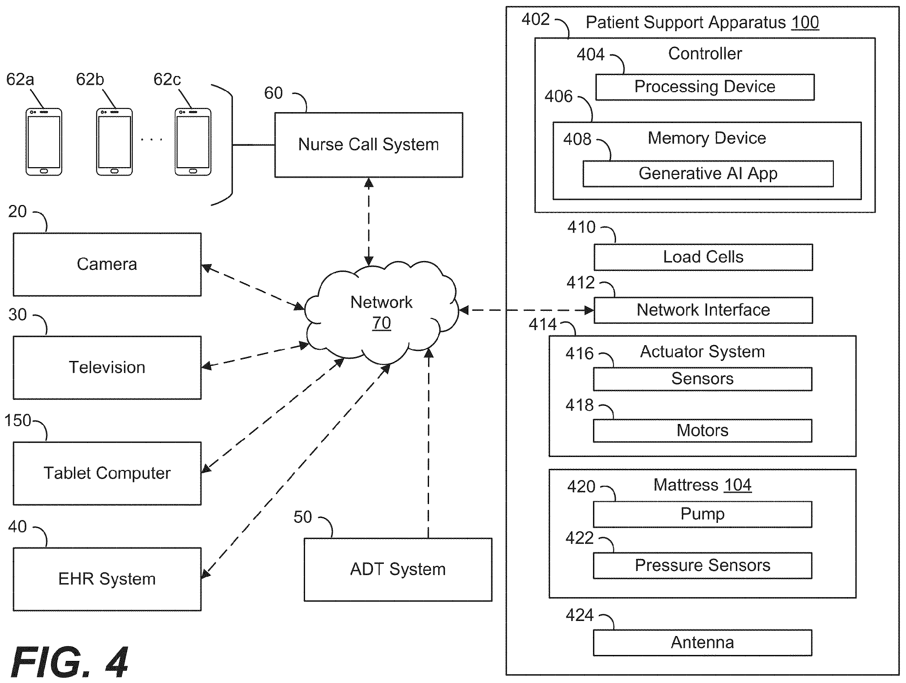

The microphone and speaker unit 124 can be used to provide voice communications between the patient P and caregivers who are not physically present near the patient support apparatus 100 via a nurse call system 60 (see FIG. 4). In some examples, the microphone and speaker unit 124 provides one-way voice communications (from the caregivers to the patient P or from the patient P to the caregivers). Alternatively, the microphone and speaker unit 124 can provide two-way voice communications between the patient P and the caregivers.

As an illustrative example, the patient P can push a button or switch on a nurse call interface 156 positioned adjacent to the microphone and speaker unit 124 on an upper siderail of the patient support apparatus 100 that causes the microphone and speaker unit 124 to record a voice message for relay to one or more devices 62 by the nurse call system 60. The microphone and speaker unit 124 can also provide playback of voice messages by the caregivers captured by the one or more devices 62. In some examples, the microphone and speaker unit 124 allows a live two-way conversation to occur between the patient P and the remotely located caregivers.

As will be further described below, a generative artificial intelligence (AI) application 408 (see FIG. 4) can utilize the microphone and speaker unit 124 to meaningfully interact with the patient P such as to identify a condition of the patient P such as a discomfort that can be relieved by adjustment of one or more settings of the patient support apparatus 100, or by providing a therapeutic therapy available on the patient support apparatus 100.

As further shown in FIGS. 1 and 2, a camera 20 captures visual data of the patient P while resting on the patient support apparatus 100. The visual data can include images, videos, and the like. The visual data captured by the camera 20 is analyzed to detect behaviors, actions, movements, and the like of the patient P which can be analyzed to detect a status or a condition of the patient P such as whether the patient P is restless or agitated. The visual data captured by the camera 90 is analyzed using artificial intelligence to identify the behaviors, actions, and movements of the patient P that are relevant to a particular status or condition of the patient P.

In some examples, the camera 20 is a pan-tilt-zoom (PTZ) camera that includes mechanical parts that allow the camera 20 to swivel left to right, tilt up and down, and zoom in and out for capturing the visual data of the patient P resting on the patient support apparatus 100. In some examples, the camera 20 can capture the visual data under different wavelengths of light such that the camera 20 can capture color images/videos, infrared images/videos, and the like.

As further shown in the example of FIGS. 1 and 2, a television 30 is mounted or positioned proximate to the patient support apparatus 100. In some examples, the television 30 is communicatively coupled to the patient support apparatus 100 such as via a wireless connection established through Bluetooth^{®}, Wi-Fi, and other wireless communications protocols. Also, the television 30 can be communicatively coupled to the patient support apparatus 100 via a wired connection such as a cable that connects the television 30 to the patient support apparatus 100.

As further shown in the example of FIGS. 1 and 2, the patient support apparatus 100 includes a patient interface device 120 that can be physically attached to the patient support apparatus 100 by an overhead arm assembly 122. The overhead arm assembly 122 allows the patient interface device 120 to be suspended in front of the patient P. The patient P can grab the patient interface device 120 to swivel the patient interface device 120 left and right and/or to pull the patient interface device 120 up and down such that the patient interface device 120 can be comfortably held by the patient P while resting on the patient support apparatus.

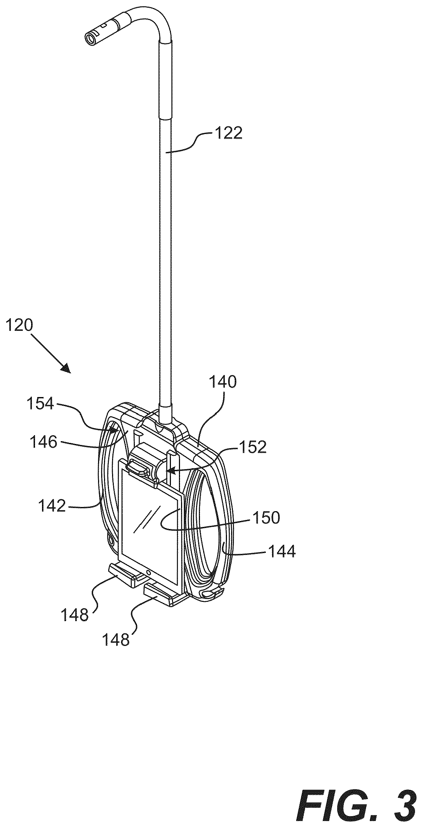

FIG. 3 is an isometric view of the patient interface device 120 that can be mounted on the patient support apparatus 100. The patient interface device 120 includes a housing 140 having handles 142, 144. The housing 140 further includes a front surface 146 having a pair of bases 148 on which a bottom of a tablet computer 150 is positioned, and an adjustable clamp 152 which engages a top of the tablet computer 150 to thereby retain the tablet computer 150 to the housing 140. The patient interface device 120 further includes a charging port 154 that can receive a first connectorized end of a cable having a second connectorized end plugged into the tablet computer 150 for charging a rechargeable battery of the tablet computer 150. In some examples, the charging port 154 further enables two-way communication between the tablet computer 150 and the patient support apparatus 100 when connected together by the cable. In some examples, the charging port 154 is a universal serial bus (USB) charging port.

The patient interface device 120 can include buttons or switches to adjust one or more settings of the patient support apparatus 100 such as to adjust an angle of a head section relative to a foot section of the bed to allow the patient P to rest on an incline. The patient support apparatus 100 can also include a patient interface 158 positioned on an upper siderail that also includes buttons or switches to adjust one or more settings of the patient support apparatus 100.

FIG. 4 schematically illustrates an example of the patient support apparatus 100. In this example, the patient support apparatus 100 is communicatively coupled via a network 70 to the camera 20, the television 30, and the tablet computer 150. The patient support apparatus 100 is also communicatively coupled via the network 70 to an electronic health record (EHR) system 40, and an admission, discharge, and transfer (ADT) system 50, and the nurse call system 60. In alternative examples, the patient support apparatus 100 can directly connect (i.e., without using the network 70) to one or more of the nurse call system 60, the ADT system 50, the EHR system 40, the tablet computer 150, the television 30, and the camera 20.

In the example embodiment shown in FIG. 4, the patient support apparatus 100 is connected to the camera 20 and to other systems and devices that capture data for detecting a condition and/or status of the patient P as well as interactions by the patient P with the patient support apparatus 100. Based on the condition, status, or interaction, the patient support apparatus 100 determines whether an adjustment to one or more settings of the patient support apparatus 100 would improve comfort and satisfaction of the patient P. As will be described in more detail further below, artificial intelligence is used to detect the interaction by the patient P with the patient support apparatus 100. Additionally, generative artificial intelligence can be used to engage the patient P by having a two-way conversation with the patient P such as to determine a cause for their behavior or movement while resting on the patient support apparatus 100. Also, artificial intelligence can be used to identify the one or more settings of the patient support apparatus 100 for adjustment to improve the comfort and satisfaction of the patient P.

The nurse call system 60 facilitates efficient interaction between the patient P resting on the patient support apparatus 100 and caregivers dispersed throughout the healthcare facility. The nurse call system 60 utilizes bedside call buttons on or adjacent to the patient support apparatus 100 such as on an upper siderail next to the microphone and speaker unit 124 or on the patient interface device 120 that allow the patient P to alert the caregivers when they need assistance while resting on the patient support apparatus 100. Once activated, the nurse call system 60 sends a signal to the nurse's station or to devices 62 carried by the caregivers while on shift in the healthcare facility to notify the caregivers of the patient's request and their location. The nurse call system 60 ensures prompt responses to medical needs of the patient while resting on the patient support apparatus 100 to improve workflow efficiency and promote patient safety.

The EHR system 40 maintains the medical history of the patient P by consolidating health information, including patient demographics, medical diagnoses, treatment plans, medications, test results, and immunization history, into a single electronic document called an electronic health record (EHR). The EHR system 40 manages a plurality of EHRs for a plurality of patients. The EHR system 40 enables sharing of data among healthcare professionals to improve coordination of care, reduce errors, and enhance patient outcomes. The EHR system 40 also supports clinical decision-making by providing real-time access to comprehensive patient information. The EHR system 40 can include features such as secure data storage and integration with other health technologies, which streamline administrative tasks, reduce paperwork, and improve overall healthcare efficiency and patient safety.

The ADT system 50 tracks and manages the patient P's journey from admission to the healthcare facility through discharge or transfer to another facility. When the patient P is first admitted, the ADT system 50 records details such as personal information, medical history, and the reason(s) for admission, while also assigning a unique identifier to the patient P. When the patient P is discharged or transferred, the ADT system 50 updates the status of the patient P, ensures accurate billing, and facilitates the transfer of relevant medical records to other departments or facilities. By automating these processes, the ADT system 50 improves the efficiency of operations in the healthcare facility, reduces errors, ensures regulatory compliance, and enhances patient care by providing up-to-date information to healthcare providers.

As shown in FIG. 4, the patient support apparatus 100 includes a controller 402. In some examples, the controller 402 is associated with the frame 102. For example, the controller 402 can be mounted on the frame 402. In other examples, the controller 402 is communicatively coupled to the frame 102 such as through one or more wired or wireless connections.

The controller 402 includes at least one processing device 404 and at least one memory device 406 that stores software instructions that, when executed by the at least one processing device 404, cause the at least one processing device 404 to perform the aspects, functions, and operations described herein. The at least one processing device 404 is an example of a processing unit such as a central processing unit (CPU). The at least one processing device 404 can include one or more CPUs. The at least one processing device 404 can include digital signal processors, field-programmable gate arrays, and/or other types of electronic circuits.

The at least one memory device 406 is an example of a computer-readable data storage device that operates to store data and instructions for execution by the at least one processing device 404. The at least one memory device 406 stores the generative AI application 408 that engages the patient P while they are resting on the patient support apparatus 100 to identify one or more settings of the patient support apparatus 100 that can be adjusted to improve the comfort of the patient P and/or the effectiveness of the healthcare provided to the patient P. The generative Al application 408 can utilize machine learning to detect interactions or patterns of interactions by the patient P with the patient support apparatus 100, which are then used to guide a conversation with the patient P to determine a reason for their behavior.

The training of the generative AI application 408 can leverage existing tools from MathWorks and Python, with the generative Al application 408 being trained using proprietary data belonging to a manufacturer of the patient support apparatus 100. While the generative AI application 408 is designed to potentially run directly on the patient support apparatus 100, current hardware limitations may cause the generative AI application 408 to be performed on external servers until hardware capabilities of the patient support apparatus 100 are enhanced.

The training architecture incorporates multiple layers of data integration and processing. The generative Al application 408 learns from various sensor inputs including load cell data that tracks patient movement and weight distribution, pressure sensor readings from mattress bladders, and positional data from bed angle sensors. This sensor fusion approach enables comprehensive monitoring of patient behavior and status.

The training of the generative AI application 408 can also focus on personalized language processing capabilities. The generative AI application 408 is designed to calibrate its language understanding for each individual patient by collecting and analyzing voice samples. This allows the generative Al application 408 to adapt its speech recognition to better understand patient-specific speech patterns and accents. Furthermore, the generative Al application 408 can be trained to adjust its speech output to match patient preferences, including matching accents or switching to different languages when appropriate.

The training methodology implements sophisticated behavioral pattern recognition by processing historical data from both individual patient interactions and larger patient populations. This includes analyzing the outcomes of previous recommendations and correlating patient conditions with specific bed settings. Inputs for training the generative Al application 408 can include outputs of other Al models whether they are discrete classification or regression AI models or other types of generative Al models. The generative Al application 408 employs closed-loop learning, continuously improving its performance based on the success rates of its recommendations and the resulting patient outcomes.

Contextual awareness can be built into the training process through the integration of multiple healthcare data sources. The generative Al application 408 learns to incorporate patient medical history from electronic health records, admission and discharge data, and nurse call system interactions. This comprehensive data integration enables the generative Al application 408 to make more informed and contextually appropriate decisions.

The neural network architecture is carefully designed with consideration given to various training techniques specifically chosen for medical applications. For example, large language models (LLMs) used to implement the generative Al application 408 can be optimized and adapted for this specific healthcare application. The training process is deliberately controlled and targeted, recognizing the stringent requirements of medical device applications.

The generative Al application 408 provides various ways to assist patient care by providing artificial intelligence driven speech to interact with the patient P based on recent observations of the patient P. The generative AI application 408 can also interact with authorized users of the patient support apparatus 100 such as registered nurses or doctors. The generative Al application 408 can answer questions from authorized users by using direct speech output from the microphone and speaker units 124 or other interface devices such as the display 134 of the control panel 130, the tablet computer 150, or the television 30. The generative Al application 408 can streamline the provision of healthcare both by addressing patient needs directly or by bringing them to the attention of caregivers in a timely manner. Further, the generative Al application 408 can understanding and process speech or other forms of inputs from the patient P in a manner that exceeds human ability, and can help mitigate staff shortages and associated costs that have become common in the healthcare industry.

The generative Al application 408 assesses the patient P's behavior based on data collected from one or more sensors that are mounted on the patient support apparatus 100 and/or that are otherwise communicatively coupled to the patient support apparatus 100. The generative Al application 408 provides improved care assistance by monitoring the status or behavior of the patient P. For example, when the generative Al application 408 detects that the patient P is restless based on the data collected from the one or more sensors, the generative Al application 408 generates a verbal output such as "Can I help you in any way?". The patient P may then choose to respond in one of many ways, and the generative AI application 408 can then navigate the patient P to find a solution to satisfy their needs. The generative Al application 408 learns about the needs of the patient P that are manifested in the behaviors exhibited by the patient P while resting on the patient support apparatus 100 from the verbal interactions with the patient P.

The generative Al application 408 can minimize bed exits or patient falls by proactively finding out what the patient P is intending to do and bringing it to an authorized user's attention before the patient P attempts to exit the patient support apparatus 100. The trigger for this workflow can include when the patient P becomes restless, goes through repeated movements, or attempts to adjust the position and/or orientation of the bed.

Another example includes monitoring movement of the patient P while resting on the patient support apparatus 100 and when there is a lack of movement from one side to the other, the generative AI application 408 can encourage the patient P to switch sides to minimize risk of pressure injury (i.e., bed sore). The generative AI application 408 can even be used to detect when the patient P is awake before instructing the patient P to switch sides.

The generative Al application 408 has the ability to listen to the patient P's requests or complaints. For example, the patient P may speak up saying that they need to use the restroom, or that they want to go for a walk. The generative Al application 408 can assist the patient P taking into consideration factors such as whether the patient P has a high falls risk (which can be identified from the electronic health record of the patient P maintained by the EHR system 40) and/or a status of the patient support apparatus 100 such as whether the siderails of the bed are locked to prevent the patient P from exiting. In some cases, the generative Al application 408 generates an alert for assistance that can be communicated via the nurse call system 60.

Additional requests that the patient P can raise while interacting with the generative Al application 408 can relate to comfort. For example, the patient P may say that the mattress 104 feels too firm (or that certain sections of the mattress 104 feel too firm) and the generative Al application 408 can then instruct the controller 402 to adjust the pressure inside one or more bladders of the mattress 104 until the patient P confirms that the mattress 104 feels comfortable. The generative Al application 408 can also make recommendations such as to adjust a positioning of the mattress 104 to improve the patient P's comfort. In some examples, the generative AI application 408 can recommend one or more activities such as a guided walk.

The generative Al application 408 can take past data into consideration either from the patient P or from a pool of patients to determine whether such adjustments to the patient support apparatus 100 and activities will meaningfully improve the patient P's stay experience.

As an illustrative example, when the patient P states that only the head section of the patient support apparatus 100 feels too firm, and based on past data the generative Al application 408 knows that in most cases this issue can be mitigated by raising the incline of the head section of the patient support apparatus 100, the generative Al application 408 can recommend raising the incline of the head section of the patient support apparatus 100. Such recommendation can also take into consideration a status of the patient support apparatus 100 such as when a feature that allows adjustment of the head section is unlocked on the patient support apparatus 100.

The generative Al application 408 can also greet the patient P with good morning when patient P wakes up and in absence of a caregiver that is bedside. During such interaction, the generative AI application 408 can acquire information such as the quality of sleep by asking "I hope you had a good night sleep?". This type of information can be used to help provide care to improve sleep quality if needed. Also, the generative AI application 408 can ask the patient P about their meal for breakfast, lunch, or dinner, and use the information to better customize meals for the patient P. Additional examples of the interactions that can occur between the generative Al application 408 and the patient P are contemplated. The information collected from such interactions can improve the stay experience and quality of healthcare provided to the patient P. Further, such information can also potentially reduce the patient P's recovery time.

The generative Al application 408 can also welcome the patient P each time they return to the patient support apparatus 100 either by directly greeting them such as with an audible welcome message output from the microphone and speaker unit 124, or by displaying a visual welcome message on the tablet computer 150 or the television 30. The welcome messages can make the patient P feel more comfortable to interact with the patient support apparatus 100 to discuss any concerns, feelings, and questions they may have during their stay. Upon receiving a communication from the patient P, the generative AI application 408 can provide assistance as requested by the patient. There may be other means to encourage the patient P to directly share their concerns, feelings, and questions with the patient support apparatus 100.

The generative Al application 408 can also compile a list of items representing patient P's status or behavior for viewing by a caregiver during the caregiver's next bedside visit. The list can either be read out to the caregiver such as by using the microphone and speaker unit 124 or by displaying the list on the display 134 mounted on the siderail of the patient support apparatus 100. The list can also be transmitted for remote processing off the patient support apparatus 100 for further processing such as by natural language processing (NLP) artificial intelligence models to further customize and improve the patient P's experience.

In certain examples, recommendations generated by the generative Al application 408 for adjusting the one or more settings of the patient support apparatus 100 can include audio outputs that can be emitted by the microphone and speaker unit 124. The recommendations can be accepted verbally by the patient P. For example, the microphone and speaker unit 124 captures audio data of the patient P for analysis to determine whether the patient P has verbally accepted the recommendation offered by the generative Al application 408.

Additionally, or alternatively, recommendations generated by the generative AI application 408 for adjusting the one or more settings of the patient support apparatus 100 can include visual outputs that can be displayed on the television 30 or on the tablet computer 150. The recommendations can be accepted on the tablet computer 150 such as by touch inputs from the patient P that are received on a touchscreen of the tablet computer 150.

The patient support apparatus 100 can access patient health information from the EHR system 40 and assess signs of health deterioration by correlating other information gathered from either sensors on the patient support apparatus 100 or adjacent devices. For example, the patient support apparatus 100 can detect signs of pneumonia for the patient P when resting on the patient support apparatus 100 when the respiratory rate of the patient P becomes abnormally high or irregular. The patient support apparatus 100 can use the detection of pneumonia to initiate a conversation with the patient P such as "I am sorry to see you go through this, you may want to raise the head section to help relax your breathing". Similarly, by gathering information about the patient P's health condition, the patient support apparatus 100 may correlate various symptoms to assess deterioration of the patient P's health and provide guidance. As another example, when the patient P has a history of depression, the patient support apparatus 100 may provide emotional support by saying something such as "I am here for you, if you are feeling anxious, let's try some breathing exercises together". In some examples, an avatar of a caregiver is displayed on the tablet computer 150 of the patient interface device 120, the television 30, or another display visible to the patient P. The avatar can be used to help the patient P breathe normally to help resolve their anxiety. The avatar of the caregiver can even offer encouragement through positive reinforcement such as generating an output through the microphone and speaker unit 124 like "You're doing a great job managing your health! Keep it up!"

The tablet computer 150 of the patient interface device 120 can be used by the patient P to communicate with the generative Al application 408 by texting. The tablet computer 150 can connect to the patient support apparatus 100 via an interface such as through Bluetooth^{®} or Wi-Fi. The generative Al application 408 can also encourage patient mobility by direct conversation. For example, when the patient support apparatus 100 detects lack of patient mobility especially on patients with risk of bed sores based on information gathered from the EHR system 40, the patient support apparatus 100 can use the generative AI application 408 to initiate a dialogue such as "It's been a while since you moved. Would you like to try some gentle stretches or sit up for a bit?" The generative AI application 408 can display the avatar of the caregiver on the tablet computer 150 encourage the patient P to follow along the beneficial exercises. The patient support apparatus 100 can also assess inputs from the camera 20 and determine any risk of patient inducing self-harm and try to mitigate the situation by kind words and positive outlook of life. The patient support apparatus 100 would then immediately try to get emergency help to minimize risk of self-induced injury. These are only a few examples and the scope of this disclosure is intended to assess various health issues and offer health advice and assist patients to help improve their experience of hospital stay as well as expedite their recovery.

The at least one memory device 406 includes computer-readable media, which includes any media that can be accessed by the at least one processing device 404. The computer-readable media can include computer-readable storage media and computer-readable communication media. The computer-readable storage media includes volatile and nonvolatile, removable and non-removable media implemented in any device that can store information such as computer-readable instructions, data structures, program modules, or other data. The computer-readable storage media can include random access memory, read only memory, electrically erasable programmable read only memory, flash memory, and other memory technology, including any medium that can be used to store information that can be accessed by the at least one processing device 404. The computer-readable storage media is non-transitory.

The computer-readable communication media embodies computer-readable instructions, data structures, program modules or other data in a modulated data signal such as a carrier wave or other transport mechanism and includes any information delivery media. The term modulated data signal refers to a signal that has one or more of its characteristics set or changed in such a manner as to encode information in the signal. The computer-readable communication media can include wired media such as a wired network or direct-wired connection, and wireless media such as acoustic, radio frequency, infrared, and other wireless media. Combinations of any of the above are within the scope of computer-readable media.

The patient support apparatus 100 includes the load cells 410 communicatively coupled to the controller 402. The load cells 410 capture data for analysis by the controller 402 to detect a center of gravity or weight distribution of the patient P while resting on the mattress 104, when the patient P changes position or moves on the mattress 104, and when the patient P exits the patient support apparatus 100, or attempts to do so.

The patient support apparatus 100 includes a network interface 412 that allows the patient support apparatus 100 to connect to the network 70. The network interface 412 can include wired interfaces and/or wireless interfaces. For example, the network interface 412 can wirelessly connect to the network 70 such as through Wi-Fi and other wireless communications protocols. Alternatively, or additionally, the network interface 412 can connect to the network 70 using wired connections such as through Ethernet or Universal Serial Bus (USB) cables.

The network 70 can include any type of wired or wireless connections or any combinations thereof. Examples of wireless connections include Wi-Fi, Bluetooth, ultra-wideband (UWB), radio frequency identification (RFID), cellular network connections, and the like. In some examples, the network 70 is an Internet-of-things (IoT) network that connects and exchanges data between one or more devices and the patient support apparatus 100 over the Internet or other communications networks. In alternative examples, one or more of the devices can directly communicate with the patient support apparatus 100 without using the network 70 such as via direct wireless or wired connections with the patient support apparatus 100.

The patient support apparatus 100 includes an actuator system 414 communicatively coupled to the controller 402. The actuator system 414 includes one or more sensors 416 mounted on the frame 102 of the patient support apparatus 100 that detect a position of the frame 102 and relative positioning of the head section, seat section, and foot section of the deck 106, which are communicated to the controller 402 to determine a state of the patient support apparatus 100 such as whether the frame 102 is in a lowered position, a raised position, a Trendelenburg position, a reverse Trendelenburg position, or other position or orientation.

The actuator system 414 further includes one or more motors 418 that can be used to adjust the positioning of the frame 102 and the relative positioning of the head, seat, and foot sections of the deck 106. The controller 402 communicates instructions to the actuator system 414 that causes the one or more motors 418 to position the frame 102 into any of the aforementioned positions based on commands received from the control panel 130 or the patient interface device 120. The one or more motors 418 can include electric motors.

The patient support apparatus 100 further includes the mattress 104 which can include a pump 420 which can be controlled by the controller 402 to inflate one or more bladders inside the mattress 104 to regulate the firmness of one or more portions of the mattress 104 such as a firmness of a portion of the mattress above the head section of the deck 106, a firmness of another portion of the mattress 104 above the seat section of the deck 106, and/or a firmness of another section of the mattress 104 above the foot section of the deck 106. In such examples, the mattress 104 is a powered air mattress that operates under different modes.

The mattress 104 can operate under a normal mode that provides continuous full-body pressure redistribution for the patient P where the pump 420 automatically adjusts the pressure inside the bladders of the mattress 104 to accommodate changes in patient weight distribution. The mattress 104 can also operate under a maximum inflate mode where the pump 420 inflates the bladders to maximize the firmness of the mattress 104. The mattress 104 can also operate under a turn mode where the pump 420 inflates one side of the mattress 104 and deflates an opposite side of the mattress 104 to facilitate turning the patient P on the mattress 104.

The mattress 104 includes pressure sensors 422 that measure the pressure inside the bladders of the mattress 104. Pressure changes inside the bladders of the mattress 104 detected by the pressure sensors 422 can be used to detect movement of the patient P on the patient support apparatus 100, in addition to, or separately from, the data captured by the load cells 410.

As further shown in FIG. 4, the patient support apparatus 100 can include an antenna 424 that detects wireless signals from objects worn or carried by the users of the patient support apparatus 100 to identify users via proximity detection technology. The wireless signals detected by the antenna 424 can include radio-frequency identification (RFID) signals, near-field communication (NFC) signals, Wi-Fi signals, and/or Bluetooth^{®} signals. The wireless signal from each object carried by a user is unique for uniquely identifying each user. In some examples, the wireless signal is emitted by a tag attached to an object worn or carried by the user such as a badge, a lanyard, a name tag, and the like. In some examples, the wireless signal is emitted by a device 62 carried by the user such as a mobile smartphone, or the like.

In further examples, identification of the users of the patient support apparatus 100 can be accomplished by analyzing the visual data captured by the camera 20 (see FIGS. 1 and 2). For example, facial recognition algorithms can be performed on the visual data captured by the camera 20 to determine an identity of a user of the patient support apparatus 100. Alternatively, or additionally, the visual data captured by the camera 20 can be analyzed to detect machine-readable data, a name, or other identification on an object worn by the user such as a badge, a lanyard, a name tag, and the like to determine the identity of the user.

In further examples, identification of the users of the patient support apparatus 100 can be accomplished by communicating with the EHR system 40 and/or the ADT system 50. For example, the EHR system 40 and/or the ADT system 50 store data that identifies which caregivers are assigned to the patient P or the room where the patient support apparatus 100 is located, which can be used for identifying the users of the patient support apparatus 100.

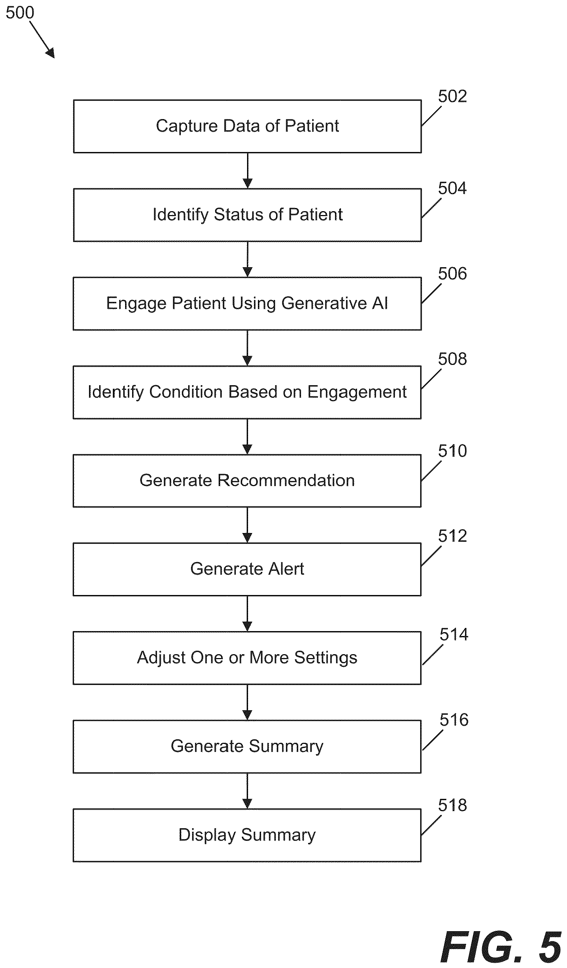

FIG. 5 schematically illustrates an example of a method 500 of providing care assistance that can be performed by the patient support apparatus 100. The method 500 enhances the effectiveness of the care assistance provided by the patient support apparatus 100.

The method 500 includes an operation 502 of capturing data of the patient P resting on the mattress 104 of the patient support apparatus 100. The data captured in operation 502 can include data captured by the load cells 410 on the patient support apparatus 100. The data captured by the load cells 410 can be analyzed to detect a center of gravity or weight distribution of the patient P while resting on the mattress 104, when the patient P changes position or moves on the mattress 104, and when the patient P exits the patient support apparatus 100, or attempts to do so. The data captured in operation 502 can also include detection of a button or switch that is pressed or selected on the patient support apparatus 100 by the patient P such as when the patient P is attempting to adjust one or more settings of the patient support apparatus 100.

Additionally, or alternatively, the data captured in operation 502 can include audio data captured by the microphone and speaker unit 124 on a siderail of the patient support apparatus 100. For example, the microphone and speaker unit 124 can perform ambient listening to detect sounds from the patient P such as from the patient P moving on the patient support apparatus 100, or from the patient P coughing, sneezing, moaning, or speaking out load.

Additionally, or alternatively, the data captured in operation 502 can include visual data of the patient P captured by the camera 20 in proximity to the patient support apparatus 100. The visual data can show the patient P moving on the patient support apparatus 100, performing actions such as sneezing, coughing, scratching, and the like. The visual data can also show the patient P attempting to use one or more features of the patient support apparatus 100.

The method 500 includes an operation 504 of identifying a status of the patient P based on the data captured in operation 502. The status of the patient P can be identified based at least in part on the motion data detected by the load cells 410 on the patient support apparatus 100. Additionally, or alternatively, the status of the patient P can be identified based at least in part on the audio data detected by the microphone and speaker unit 124. Additionally, or alternatively, the status of the patient P can be identified based at least in part on the visual data captured by the camera 20 positioned in proximity to the patient support apparatus 100.

Operation 504 can include identifying behaviors such as restlessness which can be identified from constant and repeated movements of the patient P while resting on the patient support apparatus 100. Operation 504 can include identifying lethargy such as when the patient P does not move or moves sparingly while resting on the patient support apparatus 100. Additional types of behaviors can be identified in operation 504 from the data captured in operation 502 such that the examples of restlessness and lethargy are provided for illustrative purposes.

Operation 504 can further include identifying emotional states such as anger which can be identified when the patient P bangs or hits the patient support apparatus 100 with their hands, or performs a gesture such as crossing their arms or has a facial expression such as a frown which suggests they are angry. As another example, operation 504 can further include identifying sadness such as when the patient P curls up on their side (i.e., fetal position), and/or when the audio data captured by the microphone and speaker unit 124 indicates the patient P is crying. Additional types of emotional states can be identified in operation 504 from the data captured in operation 502 such that these examples are provided for illustrative purposes.

Operation 504 can include using artificial intelligence to process and analyze the data captured in operation 502 for identifying the status including behaviors and emotional states of the patient P. The data from various sources such as the motion data captured by the load cells 410, the audio data captured by the microphone and speaker unit 124, and the visual data captured by the camera 20 can be aggregated for analysis by one or more artificial intelligence algorithms for identifying one or more behaviors and/or emotional states of the patient P.

The method 500 includes an operation 506 of engaging the patient P about their status identified in operation 504. In some examples, the engagement includes a two-way conversation driven by the generative Al application 408. As an illustrative example, when operation 504 identifies the patient P as being restless, the generative Al application 408 can emit an audible output using the microphone and speaker unit 124 such as "Can I help you in any way?". The patient P may then choose to respond in one of many ways, and the generative Al application 408 is able to adapt to the patient P's responses to navigate the conversation. Alternatively, the engagement in operation 506 can include two-way texting that can be performed on the tablet computer 150, or can include other forms of two-way communication.

Operation 506 can include acquiring information from at least one of the EHR system 40, the ADT system 50, and the nurse call system 60 to guide the engagement. For example, demographic information such as the age, sex, and ethnicity of the patient P can be acquired from one or more of these systems, and the generative AI application 408 can use the demographic information to adapt the engagement with the patient P such as by using language, slang, idioms, or even accents that can improve the patient P's understanding of the outputs of the generative Al application 408 based on their particular age, sex, and/or ethnicity.

In some examples, the generative AI application 408 calibrates the outputs based on the speech of the patient P captured by the microphone and speaker unit 124. For example, the generative Al application 408 can detect that the patient P has a certain accent that indicates English is their second language, and the generative Al application 408 can ask the patient P whether they would prefer to speak in another language such as Spanish. When the patient P responds that they prefer speaking in another language, the generative AI application 408 can adapt the outputs to be in the language preferred by the patient P.

The method 500 includes an operation 508 of identifying a condition of the patient P based on the engagement in operation 506. For example, the condition can include a discomfort felt by the patient P such as when the mattress 104 feels too firm (or certain sections of the mattress feel too firm), or the patient P would like to adjust the angle of the head section of the bed relative to the seat and foot sections. As another example, the condition can include a need or desire of the patient P such as when the patient P would like to exit the patient support apparatus 100 to toilet, to go for a walk, or to get something such as a blanket when the patient P feels cold or a cup of water when the patient P is thirsty. Additional examples are contemplated.

As another example, operation 508 can include identifying medically relevant conditions such as symptoms felt by the patient P such as pain, dizziness, nausea, and the like. In such examples, operation 508 can include storing the medically relevant conditions of the patient P to the electronic health record (EHR) of the patient P maintained by the EHR system 40.

Operation 508 can include translating a verbal conversation to text, and then analyzing the text to identify the condition of the patient P. In some examples, a large language model (LLM) analyzes the text to identify the condition of the patient P. In this manner, the condition determined from conversation with the patient P can validate or confirm a reason for the behavior of the patient P that is identified in operation 504 to improve accuracy.

The method 500 includes an operation 510 of generating a recommendation to treat the condition of the patient P. The recommendation can include an adjustment of one or more settings of the patient support apparatus 100 to treat the condition. As an illustrative example, when the patient P states during the conversation with the generative AI application 408 that mattress 104 feels too firm, the generative AI application 408 can determined that this discomfort can be mitigated by raising the head section relative to the foot section of the patient support apparatus 100. In such example, when a feature on the patient support apparatus 100 for adjusting the head section angle is unlocked, the generative AI application 408 recommends raising the head section relative to the foot section of the patient support apparatus 100.

In some examples, the generative AI application 408 can display on the television 30 or on the tablet computer 150 content such as instructions on how to adjust a setting or feature of the patient support apparatus 100. As another example, when the patient P is not a fall risk, the generative AI application 408 can recommend that the patient P go for a guided walk to improve the stay experience. The generative Al application 408 may take past data from the patient P or from a pool of patients into consideration when generating the recommendation in operation 510.

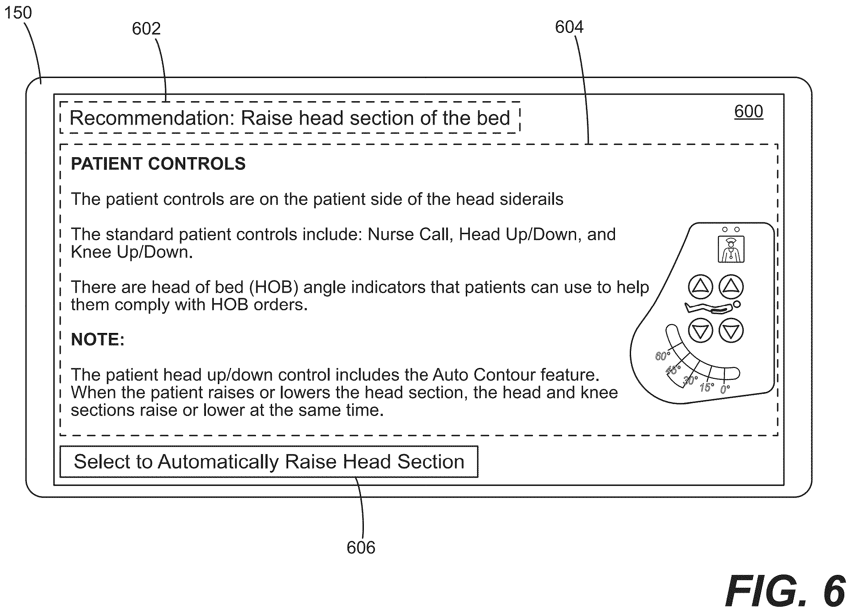

FIG. 6 illustrates the tablet computer 150 displaying an example of a graphical user interface 600 that displays a recommendation 602 generated in operation 510 of the method 500. In this illustrative example, the recommendation 602 includes instructions 604 that explain how to adjust the angle of the head section of the patient support apparatus 100 to relieve the discomfort felt by the patient P. The instructions 604 can include a diagram of the patient support apparatus 100 that identifies the location of controls for adjusting the angle of the head section.

The recommendation 602 can further include an option 606 that when selected by the patient P causes the patient support apparatus 100 to automatically implement the recommendation. For example, when option 606 is selected, the patient support apparatus 100 adjusts the angle of the head section relative to the seat and foot sections of the bed. Accordingly, when the protocols of the healthcare facility allow the patient P to adjust the settings of the patient support apparatus 100, the recommendation can include the option 606 to automatically adjust the settings of the patient support apparatus 100 without receiving prior approval from an authorized user of the patient support apparatus 100 such as a nurse. In alternative examples, the graphical user interface 600 including the recommendation 602, the instructions 604, and the option 606 can be displayed on the television 30, and the option 606 can be selected by the patient P using the remote control of the television 30.

Referring back to FIG. 5, the method 500 includes an operation 512 of generating an alert for communication to the one or more devices 62 that are remotely located relative to the patient support apparatus 100. The alert can be communicated to the one or more devices 62 by the nurse call system 60. The alert generated in operation 512 can include identification of the condition of the patient P and the recommendation to treat the condition of the patient P.

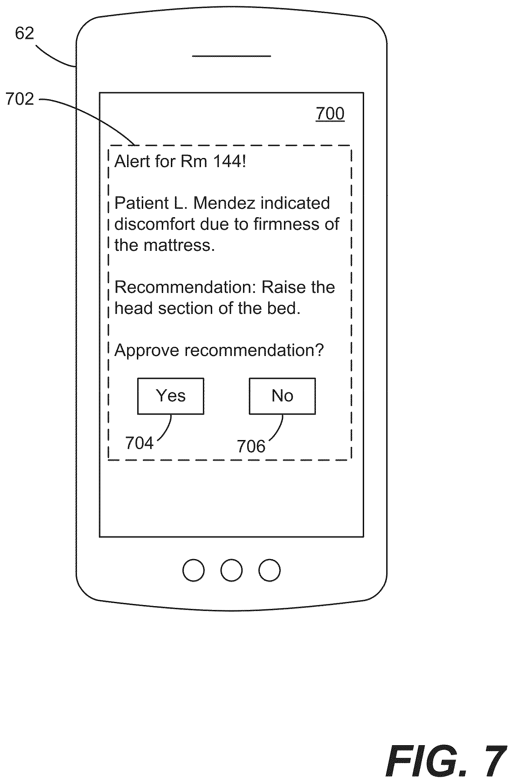

FIG. 7 illustrates a device 62 displaying an example of a graphical user interface 700 that displays an alert 702 generated in operation 512 of the method 500. As discussed above, the patient support apparatus 100 can generate the alert 702 for transmission to the device 62 via the nurse call system 60. In this illustrative example, the alert 702 identifies a location (e.g., "Room 144") of the patient support apparatus 100 and a patient (e.g., "L. Mendez") assigned to the patient support apparatus 100. The alert 702 further includes the recommendation generated in operation 510 (e.g., "Raise the head section of the bed"), a first option 704 to approve the recommendation, and a second option 706 to reject the recommendation. In alternative examples, the alert 702 including the recommendation generated in operation 510, the first option 704 to approve the recommendation, and the second option 706 to reject the recommendation can be displayed on the display 134 mounted on the siderail of the patient support apparatus 100.

Referring back to FIG. 5, the method 500 includes an operation 514 of adjusting one or more settings of the patient support apparatus 100 upon acceptance of the recommendation. Operation 514 can include adjusting a positioning of one or more of the articulable sections of the deck 106 (i.e., adjusting a relative positioning of the head section, seat section, and foot section), and/or adjusting a pressure of one or more bladders of the mattress 104, and/or performing a therapy on the patient support apparatus 100 that may include a combination of variably adjusting the positioning of one or more of the articulable sections of the deck 106 and variably adjusting the pressure of one or more bladders of the mattress 104 over a period of time.

Operation 514 can occur as a result of the patient P selecting the option 606 on the graphical user interface 600 displayed on the television 30 or tablet computer 150 to implement the recommendation. Alternatively, operation 514 can occur as a result of an authorized user selecting the first option 704 on the graphical user interface 700 displayed on the device 62 or on the display 134 mounted on the patient support apparatus 100 to approve the recommendation.

The method 500 can include an operation 516 of generating a summary of the conversation between the patient P and the generative AI application 408 from engaging the patient P in operation 506. Operation 516 can include converting the conversation into a text transcript, and then condensing the text transcript using a large language model (LLM) to generate the summary. The summary of the conversation can include highlights such as medically relevant information including symptoms, pain, or discomfort felt by the patient P, or a condition or mood of the patient P such as whether the patient P is depressed or anxious. In some examples, the summary of the conversation between the patient P and the generative Al application 408 can be stored in the EHR of the patient P maintained by the EHR system 40.

The method 500 can include an operation 518 of displaying the summary on the patient support apparatus 100 such as on the display 134 mounted on the siderail of the patient support apparatus. In some examples, the summary is displayed on the display 134 only when an authorized user is detected as being proximate to the patient support apparatus 100. Otherwise, when an authorized user is not detected as being proximate to the patient support apparatus 100, the display 134 can display a screen saver, a home screen, or can be turned off. Detection of the authorized user can be accomplished by the antenna 424 detecting a wireless signal from an object worn or carried by the authorized user to identify user via proximity detection technology.

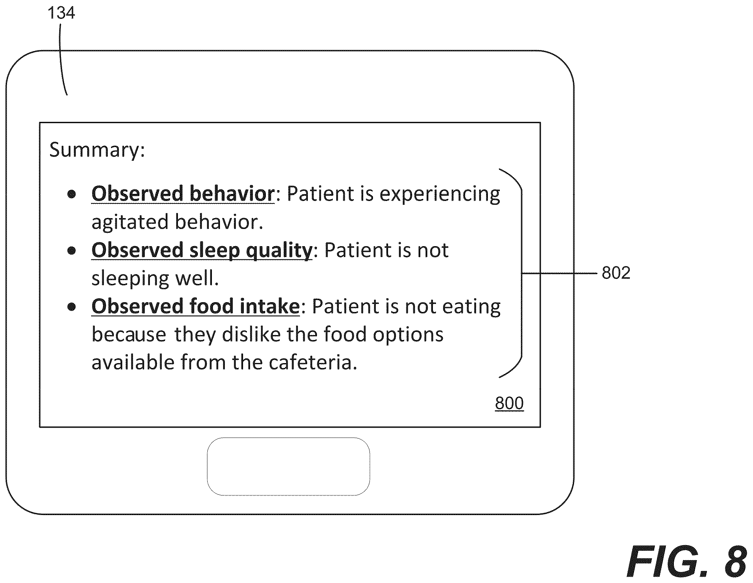

FIG. 8 illustrates the display 134 of the patient support apparatus 100 displaying an example of a graphical user interface 800 that includes a display of a summary 802 of the conversation between the patient P and the generative Al application 408 that can be generated in operation 512 of the method 500. The summary 802 can help improve the quality of healthcare provided to the patient P. For example, the summary 802 can include behavioral observations of the patient P (e.g., "patient is experiencing agitated behavior"), and can include additional observations such as sleep quality observations (e.g., "patient is not sleeping well"), and food intake observations (e.g., "patient is not eating because they dislike the food options that are available from the cafeteria") detected based on the conversation with the patient P. Such information can help guide an authorized user of the patient support apparatus 100 such as a nurse when performing scheduled rounding or when responding to a nurse call request.

Embodiments of the invention can be described with reference to the following numbered clauses, with preferred features laid out in the dependent clauses:
1 A patient support apparatus, comprising:
   a frame having one or more sensors;
   a deck coupled to the frame, the deck configured to support a mattress; and
   a controller communicatively coupled to the frame, the controller having at least one processing device, and at least one memory device storing software instructions that, when executed by the at least one processing device, cause the at least one processing device to:
      receive a communication from a patient resting on the mattress;
      engage the patient by using generative artificial intelligence;
      identify a status of the patient based on engagement with the patient; and
      generate a recommendation based on the status of the patient.
2. The patient support apparatus of clause 1, wherein the instructions, when executed by the at least one processing device, further cause the at least one processing device to:
   capture data of the patient while resting on the mattress using the one or more sensors;
   identify a behavior of the patient based on the data captured by the one or more sensors; and
   guide the engagement with the patient based on the behavior of the patient.
3. The patient support apparatus of clause 1 or 2, wherein the instructions, when executed by the at least one processing device, further cause the at least one processing device to:
   adjust one or more settings of the patient support apparatus upon acceptance of the recommendation.

## Claims

1. A patient support apparatus, comprising:
a frame having one or more sensors;
a deck coupled to the frame, the deck configured to support a mattress; and
a controller communicatively coupled to the frame, the controller having at least one processing device, and at least one memory device storing software instructions that, when executed by the at least one processing device, cause the at least one processing device to:
capture data of a patient resting on the mattress using the one or more sensors;
identify a status of the patient based on the data of the patient;
engage the patient about the status by using generative artificial intelligence; and
generate a recommendation based on engagement with the patient.

2. The patient support apparatus of claim 1, wherein the instructions, when executed by the at least one processing device, further cause the at least one processing device to:
adjust one or more settings of the patient support apparatus upon acceptance of the recommendation, wherein adjustment of the one or more settings includes at least one of adjusting a positioning of one or more articulable sections of the deck, adjusting a pressure of one or more bladders of the mattress, and performing a therapy.

3. The patient support apparatus of claim 1 or 2, wherein the one or more sensors include load cells, wherein the data of the patient includes motion data captured by the load cells, and wherein the status of the patient is identified based at least in part on the motion data.

4. The patient support apparatus of any of claims 1-3, further comprising:
a microphone and speaker unit included on a siderail of the patient support apparatus, wherein the data of the patient includes audio data captured by the microphone and speaker unit, and wherein the status of the patient is identified based at least in part on the audio data.

5. The patient support apparatus of any of claims 1-4, wherein the instructions, when executed by the at least one processing device, further cause the at least one processing device to:
receive visual data of the patient captured by a camera in proximity to the patient support apparatus, wherein the data of the patient includes visual data captured by the camera, and wherein the status of the patient is identified based at least in part on the visual data.

6. The patient support apparatus of any of claims 1-5, wherein the instructions, when executed by the at least one processing device, further cause the at least one processing device to:
generate an alert for communication to one or more devices remotely located relative to the patient support apparatus, the alert identifying the status of the patient and the recommendation.

7. The patient support apparatus of any of claims 1-6, wherein the instructions, when executed by the at least one processing device, further cause the at least one processing device to:
generate a summary of the engagement with the patient using a large language model; and
display the summary on a display mounted on the patient support apparatus.

8. The patient support apparatus of any of claims 1-7, wherein the instructions, when executed by the at least one processing device, further cause the at least one processing device to:
display an avatar for engaging with the patient.

9. The patient support apparatus of any of claims 1-8, wherein the instructions, when executed by the at least one processing device, further cause the at least one processing device to:
determine a symptom of the patient based on the engagement with the patient; and
store the symptom in an electronic health record of the patient.

10. A method of providing care assistance, the method comprising:
capturing data of a patient resting on a mattress of a patient support apparatus;
identifying a status of the patient based on the data of the patient;
engaging the patient about the status by using generative artificial intelligence;
generating a recommendation based on engagement with the patient; and
adjusting one or more settings of the patient support apparatus upon acceptance of the recommendation.

11. The method of claim 10, wherein adjustment of the one or more settings includes at least one of adjusting a positioning of one or more articulable sections of a deck, adjusting a pressure of one or more bladders of the mattress, and performing a therapy.

12. The method of claim 10 or **11,** wherein the data of the patient includes motion data captured by load cells mounted on the patient support apparatus, and wherein the status of the patient is identified based at least in part on the motion data.

13. The method of any of claims 10-12, wherein the data of the patient includes audio data captured by a microphone and speaker unit on a siderail of the patient support apparatus, and wherein the status of the patient is identified based at least in part on the audio data.

14. The method of any of claims 10-13, further comprising:
receiving visual data of the patient captured by a camera in proximity to the patient support apparatus, wherein the data of the patient includes visual data captured by the camera, and wherein the status of the patient is identified based at least in part on the visual data.

15. The method of any of claims 10-14, further comprising:
generating an alert for communication to one or more devices remotely located relative to the patient support apparatus, the alert identifying the status of the patient and the recommendation.
